# EUROPÄISCHE PATENTANMELDUNG

(11) **EP 2 108 949 A1**
(43) Veröffentlichungstag der Anmeldung: **14.10.2009**
(21) Anmeldenummer: 09009532.4
(22) Anmeldetag: 07.06.2006
(51) Int. Cl.: G01N 27/60, G01N 33/36, D01G 31/00

(54) **Verfahren und Vorrichtung zur Erkennung von Fremdstoffen in einem bewegten, festen, länglichen Prüfgut**

(30) Priorität: 15.06.2005 CH 10192005
(62) Teilanmeldung aus: 06741631.3
(71) Anmelder: Uster Technologies AG, 8610 Uster (CH)
(72) Erfinder: Ott, Philipp, 8496 Steg (CH); Schmid, Peter, 8050 Zürich (CH)

(57) **Zusammenfassung**

Die Vorrichtung (1) zur Erkennung von Fremdstoffen in einem Prüfgut (9) wie Garn, beinhaltet einen kapazitiven Sensor (2, 3) mit einem Messkondensator (2) mit einer Durchgangsöffnung (26) für das Prüfgut (9), Mittel (4) zum Anlegen einer hochfrequenten Wechselspannung an den Messkondensator (2) zwecks Beaufschlagung der Durchgangsöffnung (26) mit einem hochfrequenten elektrischen Wechselfeld und eine Detektorschaltung (6) zur analogen Detektion eines hochfrequenten Ausgangssignals des kapazitiven Sensors (2, 3). Eine Trägerfrequenz des elektrischen Wechselfeldes beträgt zwischen 1 MHz und 100 MHz. Es ist ein Sensor (2) zur Aufnahme eines vom Prüfgut (9) herrührenden, quasistatischen elektrischen Signals vorhanden. Ferner sind Auswertemittel (7) zur Auswertung des hochfrequenten Signals und des elektrostatischen Signals und zur Erkennung von Fremdstoffen vorhanden. Dadurch ergibt sich eine Verbesserung der Selektivität. Fehlansprechungen werden reduziert, und es wird eine höhere Zuverlässigkeit und Empfindlichkeit erreicht.

## Beschreibung

Die vorliegende Erfindung liegt auf dem Gebiet der Prüfung mit elektrischen Mitteln von festen, länglichen, vorzugsweise textilen Gebilden wie Kardenband, Vorgarn, Garn oder Gewebe. Sie betrifft ein Verfahren und eine Vorrichtung zur Erkennung von Fremdstoffen in einem bewegten, festen, länglichen Prüfgut, gemäss den Oberbegriffen der unabhängigen Patentansprüche. Eine bevorzugte Anwendung ist die Garnüberwachung auf einer Spinn- oder Spulmaschine.

In der Textilindustrie besteht Bedarf nach einer zuverlässigen Erkennung und eventuell Quantifizierung von Fremdstoffen wie Polypropylen in länglichen textilen Gebilden wie Garn. Zu diesem Zweck kommen häufig optische Mittel zum Einsatz. Gemäss der WO-98/33061 A1 oder der EP-1'058'112 A1 wird ein Garn mit ultraviolettem oder infrarotem Licht abgetastet. Aufgrund der Lichtabsorption können Fremdstoffe, insbesondere Polypropylen, erkannt werden. Die EP-0'399'945 A2 lehrt die Beleuchtung von Garn mit Licht zweier verschiedener Wellenlängen. So entstehen zwei Ausgangssignale, deren Differenz ermittelt wird. Fremdstoffe wie Polypropylen ergeben eine Änderung im Differenzsignal. Alle optischen Messprinzipien haben den Nachteil, dass sie Fremdstoffe nicht erkennen, welche für das verwendete Licht durchsichtig sind, dieselbe Reflektivität ("Farbe") wie das Prüfgut haben oder im Innern des Prüfguts versteckt und von aussen unsichtbar sind.

Die Unzulänglichkeiten optischer Messprinzipien können durch den Einsatz von elektrischen Mitteln umgangen werden. Aus der EP-0'924'513 A1 sind ein Verfahren und eine Vorrichtung zur kapazitiven Ermittlung von Anteilen fester Stoffe in textilem Prüfgut bekannt. Dabei wird das Prüfgut durch einen Plattenkondensator hindurch bewegt und einem elektrischen Wechselfeld ausgesetzt. Es werden dielektrische Eigenschaften des Prüfgutes ermittelt. Aus den dielektrischen Eigenschaften werden zwei elektrische Grössen ermittelt und kombiniert, wobei ein Kennwert entsteht, der von der Masse des Prüfgutes unabhängig ist. Der Kennwert wird mit einem vorausgehend ermittelten Kennwert für die betreffenden Stoffe verglichen und daraus der Anteil Fremdstoffe bestimmt. Ein Problem bei diesem Messprinzip ist seine starke Feuchtigkeitsempfindlichkeit. In einer bevorzugten Ausführungsform der in der EP-0'924'513 A1 offenbarten Vorrichtung wird versucht, durch Luftfeuchtigkeit verursachte Störsignale zu eliminieren, indem gleichzeitig mit dem eigentlichen Messkondensator ein Referenzkondensator eingesetzt wird. Dieser kann durch Zufügen einer dritten, parallel zu den beiden Messkondensatorplatten angeordneten Kondensatorplatte gebildet werden, wobei die drei Kondensatorplatten zu einer kapazitiven Brücke zusammen geschaltet werden. Diese Massnahme beseitigt aber durch Feuchtigkeitsänderungen des Prüfgutes selbst hervorgerufene Störsignale nicht. Deshalb kommen bei diesem Messprinzip zu viele Fehlansprechungen vor.

Im Verfahren und in der Vorrichtung zur Erkennung von Fremdstoffen in Garn gemäss der US-2003/0107729 A1 werden am Garn zwei Parameter erfasst und daraus zwei Signale erzeugt. Beiden Signalen werden eigene Bewertungskriterien, bspw. Grenzwerte, zugeordnet. Aus den Bewertungen zusammen kann mindestens eine bestimmte Art von Fremdstoffen ermittelt werden, die sich aus den Bewertungskriterien ergibt. In einer bevorzugten Ausführungsform betrifft einer der Parameter optische, der andere elektrische Eigenschaften des Garns.

Die US-5,654,554 A betrifft ein Verfahren und eine Vorrichtung zur Erfassung von Eigenschaften an Garn. Die Vorrichtung weist zwei Sensoren auf. Ein einfacher, kostengünstiger Sensor, z. B. ein kapazitiver Sensor, misst kontinuierlich das Garn. Ein weiterer aufwändigerer und genauer, aber langsam arbeitender Sensor, z. B. eine Kamera, wird vom einfacheren Sensor getriggert, falls jener besondere Ereignisse anzeigt. Diese Ereignisse werden vom aufwändigen Sensor gespeichert und anschliessend mit grösserem Zeitaufwand genau analysiert.

Die EP-0'301'395 A2 offenbart ein Verfahren und eine Vorrichtung zur Detektion von Oberflächenunebenheiten auf einer bewegten, elektrisch isolierenden Bahn. Ein elektrisch leitender, quer zur Bewegungsrichtung verlaufender Draht ist in die Nähe der Bahn angeordnet. Auf der Bahnoberfläche vorhandene elektrische Ladungen erzeugen ein elektrisches Signal im Draht.

Es ist auch bekannt, einen bewegten Faden zu überwachen, indem der zu überwachende Faden an einer stationären Elektrode vorbei geführt wird. Die im Faden ungleichmässig verteilten Ladungen erzeugen in der Elektrode ein Spannungssignal. Solche Sensoren zeigen z. B. die DE-32'15'695 A1, die DE-92'16'181 U1 oder die DE-195'35'177 A1. Die mit derartigen Sensoren erzeugten Signale sind unzuverlässig und führen zu vielen Fehlansprechungen.

Gemäss der CH-526'459 A5 wird ein triboelektrischer (reibungselektrischer) Wandler verwendet, um eine Fadenüberwachungseinrichtung zu schaffen, die zwei Überwachungsfunktionen ausübt: Untersuchung auf Vorhandensein/Nichtvorhandensein des ruhenden Fadens (Anwesenheitswächter) und Untersuchung auf Längsbewegung/Stillstand (Laufwächter).

Die EP-1'037'047 A1 greift das triboelektrische Messprinzip auf. Sie beschreibt ein Verfahren und eine Vorrichtung zur Detektion von Fremdmaterialien in einem bewegten textilen Material. Es wird ein Fühler zur Aufnahme von triboelektrischen Signalen vom bewegten textilen Material bereitgestellt. Das Ausgangssignal des Fühlers wird mit einem Referenzwert verglichen, der für das textile Material ohne Fremdmaterialien kennzeichnend ist. Anhand des Ergebnisses des Vergleichs wird festgestellt, ob Fremdmaterial vorhanden ist. Auch dieses Messprinzip liefert viele Fehlansprechungen, hauptsächlich hervorgerufen durch elektrostatische Entladungen.

Die beim Stand der Technik auftretenden Fehlansprechungen sind höchst unerwünscht, weil sie zu unnötigen Abstellungen des Produktionsprozesses führen. Dadurch ergeben sich beim Anwender Produktionsausfälle. Besonders dramatisch kann dies auf Spinnmaschinen sein, wo eine Abstellung ca. 10 Minuten dauert. Der wirtschaftliche Schaden vieler unnötiger Abstellungen ist enorm.

Es ist deshalb eine Aufgabe der vorliegenden Erfindung, ein Verfahren und eine Vorrichtung zur Erkennung von Fremdstoffen in einem bewegten, festen, länglichen Textilgut anzugeben, welche die obigen Nachteile nicht aufweisen. Insbesondere sollen das Verfahren und die Vorrichtung einfach bzw. platzsparend sein. Ausserdem soll eine bessere Selektivität erzielt werden. Die Fehlansprechungen gegenüber dem Stand der Technik sollen deutlich reduziert werden, bei gleich hoher oder höherer Zuverlässigkeit und Empfindlichkeit bei der Erkennung von Fremdstoffen.

Diese und andere Aufgaben werden gelöst durch das Verfahren und die Vorrichtung, wie sie in den unabhängigen Patentansprüchen definiert sind. Vorteilhafte Ausführungsformen werden in den abhängigen Patentansprüchen angegeben.

Die Erfindung vereinfacht die - insbesondere aus der EP-1'037'047 A1 - bekannten Verfahren und Vorrichtungen, indem sie auf das Abführen bereits vorhandener Ladungen vom Prüfgut und das erneute kontrollierte Aufbringen von Ladungen auf das Prüfgut verzichtet. Stattdessen setzt sie das Vorhandensein von Ladungen auf dem Prüfgut voraus und nutzt diese Ladungen aus, um anhand von ihnen Informationen über das Prüfgut bzw. eventuelle Fremdstoffanteile zu erhalten. Diese Abkehr vom bisher bekannten Messprinzip stellt eine radikale Wende in der Fremdstofferkennung dar. Sie führt zu einer Vereinfachung beim Verfahren und eine Platzersparnis bei der Vorrichtung.

Gemäss der Erfindung wird vorzugsweise sowohl ein elektrostatisches Signal - durch Ladungsmessung - als auch ein weiteres, z. B. kapazitives oder optisches, Signal vom Prüfgut aufgenommen, und beide Signale werden zur Erkennung von Fremdstoffen verwendet. Dadurch ergibt sich eine unerwartete Verbesserung der Selektivität. Als besonders vorteilhaft hat es sich erwiesen, das elektrostatische Signal laufend auszuwerten und als Auslöser (Trigger) für eine Auswertung des weiteren Signals zu verwenden. Das elektrostatische Signal liefert dann eine Grobmessung, das weitere Signal bei Bedarf eine Feinmessung. Da das elektrostatische Signal von der Feuchtigkeit kaum beeinflusst wird, entfallen durch Feuchtigkeitsschwankungen verursachte Fehlansprechungen. Das weitere Signal verfeinert die Messung und erlaubt bei Bedarf die Berücksichtigung von Einflüssen und Parametern, die das elektrostatische Signal nicht erfassen kann. So kann z. B. aufgrund des weiteren Signals zusätzlich zu Fremdstoffanteilen auch die Dicke des Prüfgutes ermittelt werden. Besonders vorteilhaft und platzsparend ist es, wenn das elektrostatische Signal und das weitere Signal von ein und demselben Sensor, z. B. einem Messkondensator, aufgenommen werden können.

Dementsprechend beinhaltet die Erfindung die Verwendung eines Sensors zur Aufnahme eines elektrostatischen Signals zur Erkennung von Fremdstoffen in einem bewegten, festen, länglichen Prüfgut. Der Sensor ist geeignet, ein elektrostatische Signal von solchen Überschussladungen aufzunehmen, die vorgängig bereits auf dem Prüfgut vorhanden waren. Diese Überschussladungen können z. B. durch Reibung an Maschinenteilen wie Ballonbegrenzer, Fadenspanner oder Führungsösen auf das Prüfgut aufgebracht werden.

Im erfindungsgemässen Verfahren zur Erkennung von Fremdstoffen in einem bewegten, festen, länglichen Prüfgut wird ein elektrostatisches Signal vom Prüfgut aufgenommen und für die Erkennung von Fremdstoffen verwendet. Das elektrostatische Signal wird dabei von solchen Überschussladungen aufgenommen, die vorgängig bereits auf dem Prüfgut vorhanden waren. In einer bevorzugten Ausführungsform wird zusätzlich ein weiteres Signal vom Prüfgut aufgenommen, und sowohl das elektrostatische Signal als auch das weitere Signal werden für die Erkennung von Fremdstoffen verwendet.

In einer anderen Variante des erfindungsgemässen Verfahrens zur Erkennung von Fremdstoffen in einem bewegten, festen, länglichen Prüfgut wird das Prüfgut einem hochfrequenten elektrischen Wechselfeld ausgesetzt. Eigenschaften des mit dem Prüfgut wechselwirkenden elektrischen Wechselfeldes werden ermittelt, indem ein hochfrequentes elektrisches Signal aufgenommen und mit einer Trägerfrequenz des elektrischen Wechselfeldes demoduliert wird. Es wird ein zweites, vom Prüfgut herrührendes, quasistatisches elektrisches Signal aufgenommen. Sowohl das erste als auch das zweite elektrische Signal werden für die Erkennung von Fremdstoffen verwendet.

Die erfindungsgemässe Vorrichtung zur Erkennung von Fremdstoffen in einem bewegten, festen, länglichen Prüfgut beinhaltet einen Sensor zur Aufnahme eines elektrostatischen Signals vom Prüfgut sowie Auswertemittel zur Auswertung des elektrostatischen Signals und zur Erkennung von Fremdstoffen. Der Sensor ist geeignet, ein elektrostatische Signal von solchen Überschussladungen aufzunehmen, die vorgängig bereits auf dem Prüfgut vorhanden waren. In einer bevorzugten Ausführungsform beinhaltet die erfindungsgemässe Vorrichtung ferner einen Sensor zur Aufnahme eines weiteren Signals vom Prüfgut sowie Auswertemittel zur Auswertung des elektrostatischen Signals und des weiteren Signals und zur Erkennung von Fremdstoffen.

Unter einem "elektrostatischem Signal" wird in dieser Schrift ein Signal verstanden, das von auf dem Prüfgut befindlichen, dort ruhenden Ladungen herrührt. Diese Ladungen können durch galvanischen Kontakt abgegriffen oder kontaktlos, bspw. durch Influenz, detektiert werden. Im ersteren Fall fliessen zwar Ladungen, aber ihre Beschleunigungen und Geschwindigkeiten sind relativ klein, so dass immer noch von einem elektrostatischen Signal gesprochen werden kann. Beide Fälle könnten alternativ mit dem Begriff "passive Ladungsverschiebung" beschrieben werden - im Gegensatz zur aktiven Erzeugung eines hochfrequenten elektrischen Wechselfeldes, wie es in EP-0'924'S13 A1 gelehrt wird. Nachfolgend wird eine bevorzugte Ausführungsform der Erfindung anhand der beiliegenden Zeichnungen detailliert erläutert. Dabei zeigen schematisch:
- Figur 1: ein elektrisches Schaltschema einer Ausführungsform der erfindungsgemässen Vorrichtung und
- Figur 2: ein Flussdiagramm einer Ausführungsform des erfindungsgemässen Verfahrens.

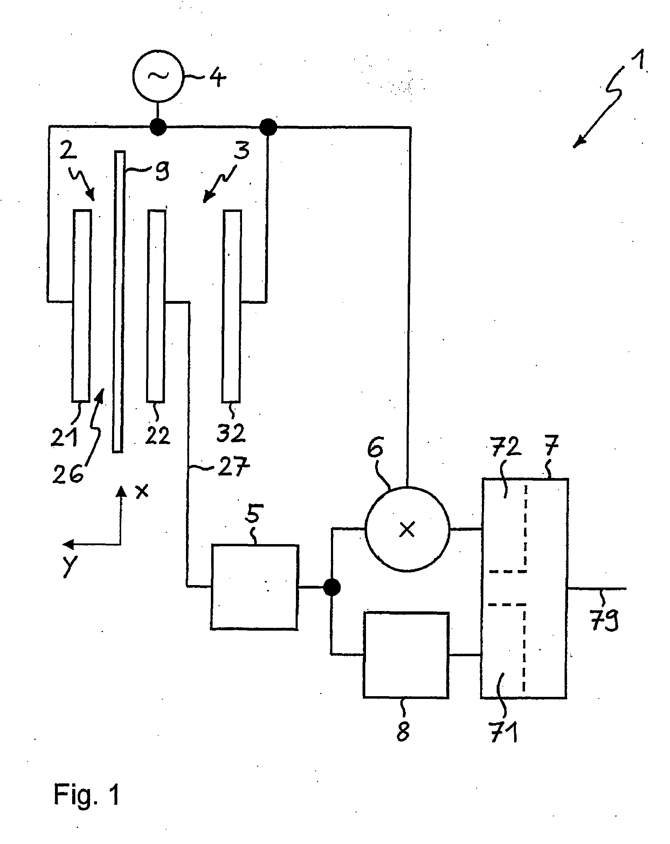

Ein elektrisches Schaltschema einer erfindungsgemässen Vorrichtung 1 ist in Figur 1 angegeben. Die Vorrichtung 1 beinhaltet einen Messkondensator 2. Dabei handelt es sich in diesem Ausführungsbeispiel um einen ebenen Zweiplattenkondensator mit einer ersten, im Wesentlichen ebenen Kondensatorplatte 21 und einer zweiten, im Wesentlichen ebenen Kondensatorplatte 22. Die Kondensatorplatten 21, 22 sind je ca. 0.8 mm dick, bestehen z. B. aus Messing und können zur Erzielung einer höheren Abriebfestigkeit z. B. mit Nickel beschichtet sein. Die beiden Kondensatorplatten 21, 22 sind durch einen ca. 1-3 mm, vorzugsweise ca. 1.5-2.0 mm dicken Luftspalt voneinander getrennt, der eine Durchgangsöffnung 26 für ein festes, längliches Prüfgut 9 bildet. Das Prüfgut 9 kann z. B. ein Garn sein. Es wird vorzugsweise in Längsrichtung x durch die Durchgangsöffnung 26 hindurch bewegt.

Es ist bekannt, dass sich textile Materialien wie das Garn 9 und andere Materialien elektrostatisch aufladen können. Eine solche Aufladung kann z. B. durch Reibung an Maschinenteilen wie Ballonbegrenzer, Fadenspanner oder Führungsösen zustande kommen. Polypropylen, ein unerwünschter Fremdstoff, den zu detektieren ein bestehendes Bedürfnis der Textilindustrie ist, lädt sich bspw. negativ auf, d. h. in einer Schicht an der Oberfläche des Polypropylens entsteht ein Elektronenüberschuss. Der Messkondensator 2 eignet sich nun zur Detektion derartiger Ladungen. Das elektrische Feld der Überschussladungen verursacht nämlich eine Potenzialänderung auf den Kondensatorplatten 21, 22, sobald die Ladungen in die Durchgangsöffnung 26 gelangen. Diese Potenzialänderung kann mittels einer Ausgangsleitung 27, die bspw. mit der zweiten Kondensatorplatte 22 verbunden ist, als elektrostatisches Signal vom Garn 9 aufgenommen werden.

Alternativ zur kontaktlosen Signalaufnahme ist es möglich, das elektrostatische Signal mittels einer (nicht dargestellten) Kontaktelektrode, die in galvanischem Kontakt mit dem Garn steht, aufzunehmen. Diese Alternative kann den Vorteil haben, empfindlicher zu sein. Ihre Nachteile sind aber der unvermeidliche Abrieb von Elektrode und Garn, der die Vorrichtung verschmutzt und die Elektrode abnützt, und der bei hohen Garngeschwindigkeiten nur schwer sicherzustellende ständige Kontakt. Schon deshalb erscheint die kontaktlose Signalaufnahme als vorteilhafter. Ein weiterer Grund, den Messkondensator 2 für die Aufnahme des elektrostatischen Signals zu verwenden, ist die Verwendbarkeit desselben Messkondensators 2 zur Aufnahme eines weiteren, kapazitiven Signals, wie nachfolgend erläutert wird.

In einer bevorzugten Ausführungsform der vorliegenden Erfindung wird ein weiteres Signal vom Garn 9 aufgenommen. In der beispielhaften, nicht einschränkenden Ausführungsform von Fig. 1 ist das weitere Signal ein kapazitives Signal. Das Garn 9 wird im Messkondensator 2 einem hochfrequenten elektrischen Wechselfeld ausgesetzt, und es werden Eigenschaften des mit dem Garn 9 wechselwirkenden elektrischen Wechselfeldes ermittelt.

Um durch äussere Einflüsse wie Lufttemperatur oder Luftfeuchtigkeit verursachte Störsignale zu verringern, beinhaltet die Ausführungsform von Fig. 1 nebst dem Messkondensator 2 einen Referenzkondensator 3 mit zwei ebenen Kondensatorplatten 22, 32. Dabei ist die mittlere Kondensatorplatte 22 beiden Kondensatoren 2, 3 gemeinsam.

Verfahren und Vorrichtungen zur kapazitiven Erkennung und Quantifizierung von festen Fremdstoffen in textilem Prüfgut 9 sind aus der EP-0'924'513 A1, insbesondere aus den Absätzen [0022] - [0034] daraus, bekannt und können auch für die vorliegende Erfindung übernommen werden. Durch diese Bezugnahme auf die EP-0'924'513 A1 erübrigt sich hier eine detaillierte Beschreibung der Auswertemethoden. Dazu sei hier nur soviel gesagt, das mindestens zwei Messmodi möglich sind. In einem ersten Messmodus wird mit zwei verschiedenen Anregungs- oder Trägerfrequenzen des elektrischen Wechselfeldes gemessen. Die beiden gleichartigen Ausgangssignale, z. B. gemessene Spannungen, werden zunächst für jede der Trägerfrequenzen separat detektiert und dann zur Auswertung auf geeignete Weise miteinander kombiniert. In einem zweiten Messmodus wird bei einer einzigen Trägerfrequenz gemessen, als Ausgangssignale jedoch Ausgangsspannung und Ausgangsstrom verwendet. Die Phasenverschiebung zwischen dem Spannungs- und dem Stromsignal liefert nach geeigneter Auswertung die gesuchte Information über das Garn 9. Auch eine Kombination der beiden Messmodi, d. h. Messung bei mehreren Trägerfrequenzen und Messung der jeweiligen Phasenverschiebungen zwischen Spannungs- und Stromsignal, ist möglich.

Zum Zweck der kapazitiven Signalaufnahme beinhaltet die erfindungsgemässe Vorrichtung 1 einen Wechselspannungsgenerator 4 zum Anlegen einer hochfrequenten Wechselspannung an den Messkondensator 2 und den Referenzkondensator 3. Unter "Hochfrequenz" wird hier gemäss dem in der Elektrotechnik gängigen Sprachgebrauch eine Frequenz zwischen 100 kHz und 300 GHz verstanden. Vorzugsweise beträgt die Frequenz der angelegten Wechselspannung zwischen 1 MHz und 1.00 MHz, z. B. 10 MHz. Somit kann gesagt werden, dass ein kapazitiver Spannungsteiler mit zwei Kondensatoren 2, 3 vorliegt, der durch das Prüfgut 9 verstimmt werden kann. Mit der Ausgangsleitung 27 der Kondensatoren 2, 3 ist vorzugsweise ein Impedanzwandler 5 verbunden. Der Impedanzwandler 5 kann z. B. als Kollektorschaltung oder als Transimpedanz-Verstärker-Schaltung mit einem Operationsverstärker ausgebildet sein. Dem Impedanzwandler 5 nachgeschaltet ist eine Detektorschaltung 6 zur analogen Detektion des Ausgangssignals der Kondensatoren 2, 3. Im Ausführungsbeispiel von Figur 1 führt die Detektorschaltung 6 im Wesentlichen eine Multiplikation des Ausgangssignals des Messkondensators 2 mit dem an den Kondensatoren 2, 3 angelegten Wechselspannungssignal aus, um es zu demodulieren. Das demodulierte kapazitive Messsignal wird einer Auswerteschaltung 7 zugeführt.

Im Gegensatz zum hochfrequenten kapazitiven Signal ist das elektrostatische Signal quasistatisch. Es kann mit einer Bandbreite von bspw. 5 bis 10 kHz detektiert werden. Um das quasistatische Signal aus dem hochfrequenten Signal zu extrahieren, wird bspw. ein Spitzenwertgleichrichter 8 verwendet, der mit der Ausgangsleitung 27 des Messkondensators 2 elektrisch verbunden und vorzugsweise dem Impedanzwandler 5 nachgeschaltet ist. Das Ausgangssignal des Spitzenwertgleichrichters 8 wird ebenfalls der Auswerteschaltung 7 zugeführt.

Die Auswerteschaltung 7 ermittelt aus dem elektrostatischen Signal und dem kapazitiven Signal das eigentliche Resultat, nämlich die Fremdstoffanteile im Garn 9, und gibt ein Ausgangssignal auf einer Ausgangsleitung 79 der Vorrichtung aus. Es ist möglich, den quantitativen Anteil der Fremdstoffe und allenfalls das Material der Fremdstoffe, daneben auch andere Parameter wie die Garndicke zu bestimmen. Die Auswerteschaltung 7 kann als analoge elektrische Schaltung oder als digitaler Prozessor ausgebildet sein. Sie kann Teil eines anwenderspezifischen integrierten Schaltkreises (ASIC) sein, auf dem ebenfalls andere Elemente wie der Impedanzwandler 5, die Detektorschaltung 6 und/oder der Spitzenwertgleichrichter 8 integriert sein können. Die Auswerteschaltung 7 kann ein erstes Modul 71 für die Auswertung des elektrostatischen Signals und ein zweites Modul 72 für die Auswertung des weiteren Signals beinhalten.

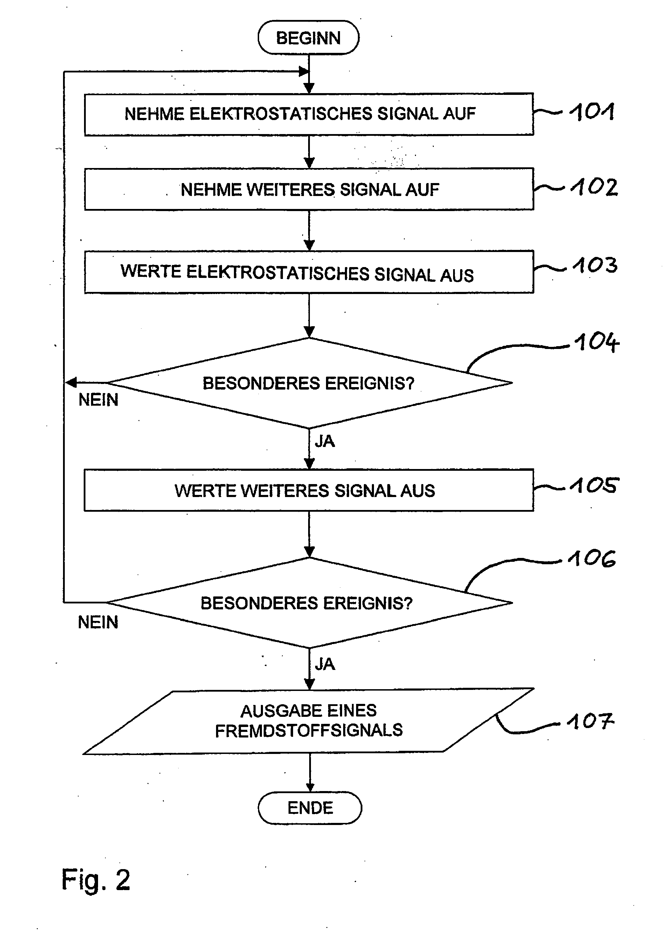

Figur 2 zeigt schematisch ein Flussdiagramm einer bevorzugten Ausführungsform des erfindungsgemässen Verfahrens. Demgemäss werden ein elektrostatisches Signal vom Prüfgut 9 aufgenommen 101, und zwar von solchen Überschussladungen, die vorgängig bereits auf dem Prüfgut vorhanden waren. Es wird ein weiteres Signal vom Prüfgut aufgenommen 102. Das weitere Signal kann ein kapazitives Signal sein. Die Aufnahme 101 des elektrostatischen Signals kann mittels der in Fig. 1 dargestellten Vorrichtung 1 und insbesondere durch den Messkondensator 2, den Impedanzwandler 5 sowie den Spitzenwertgleichrichter 8 erfolgen. Die Aufnahme 102 des weiteren Signals kann mittels der in Fig. 1 dargestellten Vorrichtung 1 und insbesondere durch den Messkondensator 2, den Impedanzwandler 5 sowie die Detektorschaltung 6 erfolgen.

Das elektrostatische Signal wird laufend ausgewertet 103, bspw. im ersten Modul 71 der Auswerteschaltung 7 (siehe Fig. 1). Das Resultat der Auswertung 103 wird laufend auf ein besonderes Ereignis hin überprüft 104. Was als "besonderes Ereignis" eingestuft wird, hängt von der jeweiligen Anwendung ab; es kann eine Abweichung von einem Sollwert, ein Über- oder Unterschreiten eines Grenzwertes, eine plötzliche Signaländerung, eine Signaländerung, deren Ableitung einen vorgegebenen Wert über- oder unterschreitet, oder ein anderes Charakteristikum des Auswertungsresultates sein.

Die Feststellung 104 eines besonderen Ereignisses löst die Auswertung 105 des weiteren Signals aus, bspw. im zweiten Modul 72 der Auswerteschaltung 7 (siehe Fig. 1). Das Resultat dieser Auswertung 105 wird nun ebenfalls auf ein besonderes Ereignis hin überprüft 106, wobei dieses nach denselben oder anderen Kriterien definiert sein kann als das besondere Ereignis 104 beim elektrostatischen Signal. Wird kein besonderes Ereignis festgestellt 106, so deutet dies auf eine Fehlanzeige des elektrostatischen Signals hin, z. B. wegen elektrostatischer Entladungen, und die Signalaufnahme 101, 102 wird fortgesetzt. Zeigt hingegen auch die Auswertung 105 des weiteren Signals ein besonderes Ereignis an 106, so liegt mit grosser Wahrscheinlichkeit im betreffenden Garnabschnitt eine Verunreinigung durch Fremdstoffe vor. Diesfalls gibt die Auswerteeinheit 7 ein Fremdstoffsignal auf der Ausgangsleitung 79 aus 107. Das Fremdstoffsignal kann ein einfaches Signal sein, das im Sinne einer binären Ja/Nein-Entscheidung einen Garnschnitt auslöst, einen Zählschritt aktiviert, einen Alarm auslöst oder zu einer Maschinenabstellung führt. Es kann aber auch Information über Art und/oder Menge der detektierten Fremdstoffe bzw. Fremdstoffanteile enthalten. Derartige Fremdstoffanteile können von der Auswerteeinheit 7 aufgrund der Auswertungen beider Signale ermittelt werden. Selbstverständlich kann während und/oder nach dem Ausgabeschritt 107 die Signalaufnahme 101, 102 fortgesetzt werden, was der Einfachheit halber in Fig. 2 nicht dargestellt ist.

Selbstverständlich ist die Erfindung nicht auf die oben beschriebenen Ausführungsformen beschränkt. So ist es z. B. nicht nötig, für die Messung des weiteren Signals Messkondensatoren 2 mit ebenen Kondensatorplatten zu verwenden; andere Kondensatorformen kommen auch in Frage. Das weitere Signal kann elektrischer, optischer oder anderer Art sein. Die Reihenfolge der Verfahrensschritte muss nicht notwendigerweise derjenigen von Fig. 2 entsprechen; die Aufnahme 102 des weiteren Signals könnte z. B. erst nach der Feststellung 104 eines besonderen Ereignisses im elektrostatischen Signal erfolgen. Ebenso ist es möglich, zuerst das weitere Signal 106 und erst danach das elektrostatische Signal 104 auf ein besonderes Ereignis hin zu überprüfen. Die Signalaufnahmen 101, 102, die Signalauswertungen 103, 105 und/oder die Überprüfungen 104, 106 können jeweils auch gleichzeitig erfolgen.

### Bezugszeichenliste:

- 1: Vorrichtung

- 2: Messkondensator
- 21: erste Kondensatorplatte
- 22: zweite Kondensatorplatte
- 26: Durchgangsöffnung
- 27: Ausgangsleitung

- 3: Referenzkondensator
- 32: Kondensatorplatte

- 4: Wechselspannungsgenerator

- 5: Impedanzwandler

- 6: Detektorschaltung

- 7: Auswerteschaltung
- 71, 72: erstes bzw. zweites Modul der Auswerteschaltung

- 79: Ausgangsleitung der Vorrichtung

- 8: Spitzenwertgleichrichter

## Patentansprüche

1. Verfahren zur Erkennung von Fremdstoffen in einem bewegten, festen, länglichen Prüfgut (9), wobei das Prüfgut (9) einem hochfrequenten elektrischen Wechselfeld ausgesetzt wird und Eigenschaften des mit dem Prüfgut (9) wechselwirkenden elektrischen Wechselfeldes ermittelt werden, indem ein hochfrequentes elektrisches Signal aufgenommen wird (102),
**dadurch gekennzeichnet, dass**
eine Trägerfrequenz des elektrischen Wechselfeldes zwischen 1 MHz und 100 MHz, vorzugsweise ca. 10 MHz, beträgt,
ein zweites, vom Prüfgut (9) herrührendes, quasistatisches elektrisches Signal aufgenommen (101) wird und
sowohl das erste als auch das zweite elektrische Signal für die Erkennung von Fremdstoffen verwendet werden.

2. Verfahren nach Anspruch 1, wobei das zweite elektrische Signal von solchen Überschussladungen aufgenommen wird, die vorgängig bereits auf dem Prüfgut (9) vorhanden waren.

3. Verfahren nach einem der vorangehenden Ansprüche, wobei das quasistatische Signal laufend ausgewertet wird (103) und Resultate seiner Auswertung verwendet werden (104), um eine Auswertung (105) des hochfrequenten Signals auszulösen.

4. Verfahren nach einem der vorangehenden Ansprüche, wobei als Eigenschaften des mit dem Prüfgut (9) wechselwirkenden elektrischen Wechselfeldes zwei elektrische Grössen ermittelt und derart miteinander kombiniert werden, dass ein Kennwert entsteht, der von der Masse des Prüfgutes (9) unabhängig ist.

5. Verfahren nach Anspruch 4, wobei die zwei ermittelten elektrischen Grössen zwei mit zwei verschiedenen Trägerfrequenzen demodulierte Spannungssignale und/oder ein mit einer Trägerfrequenz demoduliertes Spannungs- und ein mit derselben Trägerfrequenz demoduliertes Stromsignal sind.

6. Verfahren nach einem der vorangehenden Ansprüche, wobei ein und derselbe Sensor, vorzugsweise ein Messkondensator (2), für die Aufnahme des ersten (102) und des zweiten (101) elektrischen Signals verwendet wird.

7. Vorrichtung (1) zur Erkennung von Fremdstoffen in einem bewegten, festen, länglichen Prüfgut (9), beinhaltend einen kapazitiven Sensor (2, 3) mit einem Messkondensator (2) mit einer Durchgangsöffnung (26) für das Prüfgut (9), Mittel (4) zum Anlegen einer hochfrequenten Wechselspannung an den Messkondensator (2) zwecks Beaufschlagung der Durchgangsöffnung (26) mit einem hochfrequenten elektrischen Wechselfeld und
eine Detektorschaltung (6) zur analogen Detektion eines hochfrequenten Ausgangssignals des kapazitiven Sensors (2, 3),
**dadurch gekennzeichnet, dass**
eine Trägerfrequenz des elektrischen Wechselfeldes zwischen 1 MHz und 100 MHz, vorzugsweise ca. 10 MHz, beträgt,
ein Sensor (2) zur Aufnahme eines vom Prüfgut (9) herrührenden, quasistatischen elektrischen Signals vorhanden ist sowie
Auswertemittel (7) zur Auswertung des hochfrequenten Signals und des elektrostatischen Signals und zur Erkennung von Fremdstoffen vorhanden sind.

8. Vorrichtung (1) nach Anspruch 7, wobei der zweite Sensor (2) geeignet ist, ein elektrostatisches Signal von solchen Überschussladungen aufzunehmen, die vorgängig bereits auf dem Prüfgut (9) vorhanden waren.

9. Vorrichtung (1) nach Anspruch 7 oder 8, wobei der Sensor (2) zur Aufnahme eines elektrostatischen Signals und der kapazitive Sensor (2, 3) in ein und demselben Sensor (2, 3) verwirklicht sind.

10. Vorrichtung (1) nach einem der Ansprüche 7-9, wobei die Detektorschaltung (6) geeignet ist, im Wesentlichen eine Multiplikation des Ausgangssignals des Messkondensators (2) mit dem am Messkondensator (2) angelegten Wechselspannungssignal auszuführen, um es zu demodulieren.
